(19) Europäisches Patentamt
European Patent Office
Office européen des brevets

(11) Veröffentlichungsnummer: **0 017 952**
**B1**

(12) # EUROPÄISCHE PATENTSCHRIFT

(45) Veröffentlichungstag der Patentschrift: **22.12.82**

(21) Anmeldenummer: **80101995.1**

(22) Anmeldetag: **14.04.80**

(51) Int. Cl.³: **C 07 C 69/747,** C 07 C 67/343, C 07 C 43/225, C 07 C 41/22, C 07 C 41/30, C 07 C 43/23, C 07 D 317/48 // C07F9/40, C07C45/45, C07C47/277

(54) **Verfahren zur Herstellung von Styryl-cyclopropan-carbonsäureestern, Zwischenprodukte dafür und Verfahren zu deren Herstellung.**

(30) Priorität: **23.04.79 DE 2916343**

(43) Veröffentlichungstag der Anmeldung: **29.10.80 Patentblatt 80/22**

(45) Bekanntmachung des Hinweises auf die Patenterteilung: **22.12.82 Patentblatt 82/51**

(84) Benannte Vertragsstaaten: **BE CH DE FR GB IT LI NL**

(56) Entgegenhaltungen: **„Chemical abstracts", Bd. 85, 1976, Zusammenfassung Nr. 46058h, S. 481, Columbus, Ohio, US, und JP-A Nr. 7619747 (Sumitomo Chemical Co.Ltd.) (17-02-1976)**

(73) Patentinhaber: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Harnisch, Horst, Dr., Heinenbusch 4, D-5203 Much (DE)**
Erfinder: **Lantzsch, Reinhard, Dr., Heymannstrasse 32, D-5090 Leverkusen (DE)**
Erfinder: **Naumann, Klaus, Dr., Richard-Wagner-Strasse 9, D-5090 Leverkusen 1 (DE)**
Erfinder: **Riebel, Hans-Jochem, Dr., In der Beek 92, D-5600 Wuppertal 1 (DE)**
Erfinder: **Schröder, Rolf, Dr., Pahlkestrasse 17, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Styrylcyclopropancarbonsäureestern, Zwischenprodukte dafür und Verfahren zu deren Herstellung

Die Erfindung betrifft ein neues Verfahren zur Herstellung von Styrylcyclopropancarbonsäureestern, und Zwischenprodukte zur Durchführung dieses Verfahrens sowie deren Herstellung.

Es ist bekannt, dass man bestimte 3-(2-Chloro-2-arylvinyl)-2,2-dimethylcyclopropan-1-carbonsäureester, wie z.B. 3-(2-Chloro-2-phenylvinyl)-2,2-dimethylcyclopropan-1-carbonsäureäthylester erhält, wenn man entsprechende Arylmethanphosphonsäureester mit einer starken Base, wie z.B. Butyllithium, behandelt, und dann nach einander Tetrachlorkohlenstoff und 3-Formyl-2,2-dimethylcyclopropan-1-carbonsäureester dazu gibt (vgl. DE-OS Nr. 2738150).

Aus JP-A Nr. 7619747 ist bekannt, 1,1-Dichlor-4-methyl-1,3-pentadien mit Diazoessigestern zu 3-Dichlorvinyl-2,2-dimethylcyclopropancarbonsäureestern umzusetzen. Es ist daraus jedoch nichts über Styrylcyclopropancarbonsäureester bekannt.

Dieses Herstellungsverfahren für 3-(2-Chloro-2-arylvinyl)-2,2-dimethylcyclopropan-1-carbonsäureester weist jedoch eine Reihe von Nachteilen auf:

Die zur Deprotonierung der Arylmethanphosphonsäureester benötigten starken Basen, wie z.B. Butyllithium, sind feuchtigkeits- und luftempfindlich; daher wird das konventionelle Verfahren in einer Inertgasatmosphäre, beispielsweise unter Stickstoff oder Argon und unter Verwendung eines inerten, sorgfältig getrockneten Lösungs- oder Verdünnungsmittels durchgeführt. Da die Umsetzung bei tiefer Temperatur, etwa bei −70° C, durchzuführen ist, muss das Reaktionsgemisch auch intensiv gekühlt werden. Die Aufarbeitung des Reaktionsgemisches zur Isolierung der gewünschten Produkte, welche man als Isomerengemische erhält, ist ebenfalls aufwendig; sie umfasst mehrere Extraktionen und Lösungsmitteldestillationen sowie einen chromatographischen Trennungsprozess. Die oben erläuterte bekannte Synthesemethode dürfte daher für eine Herstellung der 3-(2-Chloro-2-arylvinyl)-2,2-dimethylcyclopropan-1-carbonsäureester im industriellen Massstab wenig geeignet sein und sollte möglichst durch ein einfacher durchführbares Verfahren ersetzt werden.

Gegenstand der vorliegenden Erfindung sind

1) ein Verfahren zur Herstellung von Styrylcyclopropancarbonsäureestern der Formel I

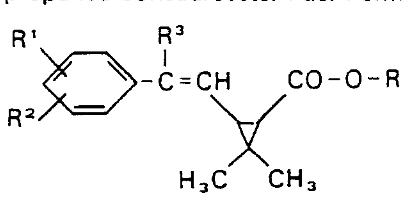 (I)

in welcher

R für $C_1$ bis $C_4$-Alkyl oder für einen bei Pyrethroiden üblichen Alkoholrest steht,

$R^1$ für Alkoxy oder Alkylthio steht, die gegebenenfalls durch Halogen substituiert sind,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht,

dadurch gekennzeichnet, dass man 1-Aryl-4-methylpenta-1,3-diene der Formel II

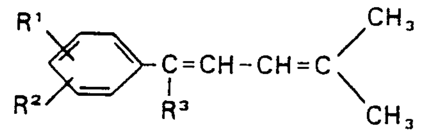 (II)

in welcher

$R^1$, $R^2$, $R^3$ die oben angegebene Bedeutung haben,

mit Diazoessigsäureestern der Formel III

$$N_2CH-COO-R \qquad (III)$$

in welcher

R die oben angegebene Bedeutung hat, in Gegenwart von Kupfer oder Kupferverbindungen bei Temperaturen zwischen 50 und 200° C umsetzt;

2) neue 1-Aryl-4-methylpenta-1,3-diene der Formel II (oben), worin

$R^1$, $R^2$ und $R^3$ die oben angegebene Bedeutung haben;

3) ein Verfahren zur Herstellung von 1-Aryl-4-methylpenta-1,3-dienen der Formel II (oben), worin

$R^1$ und $R^2$ die oben angegebene Bedeutung haben, und

$R^3$ für Halogen steht,

das dadurch gekennzeichnet ist, dass man 3-Methylbuten-2-yl-arylketone der Formel IVa

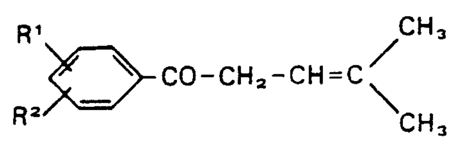 (IVa)

und/oder die dazu isomeren 4-Methylbuten-1-yl-arylketone der Formel IVb

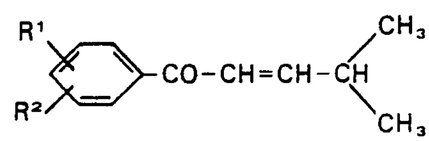 (IVb)

worin

$R^1$ und $R^2$ die oben angegebene Bedeutung haben, mit Phosphorpentahalogenid, gegebenenfalls unter Verwendung eines inerten Verdünnungsmittels, bei Temperaturen zwischen −40° C und +30° C umsetzt, und anschliessend das Reaktionsgemisch im gleichen Temperaturbereich mit einem Säurebindemittel behandelt, gegebenenfalls in Gegenwart eines Katalysators;

4) ein Verfahren zur Herstellung von 1-Aryl-4-methylpenta-1,3-dienen der Formel II (oben), worin

$R^1$, $R^2$ und $R^3$ die oben angegebene Bedeutung haben,

das dadurch gekennzeichnet ist, dass man Arylmethanphosphonsäureester der Formel V

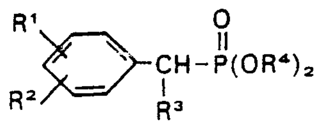 (V)

in welcher

$R^1$, $R^2$ und $R^3$ die oben angegebene Bedeutung haben und

$R^4$ für Alkyl oder Phenyl steht oder worin die Reste $R^4$ zusammen für geradkettiges oder verzweigtes Alkandiyl stehen,

mit Dimethylacrolein der Formel VI

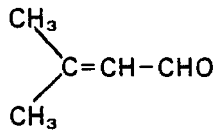 (VI)

in Gegenwart einer Base und gegebenenfalls unter Verwendung eines Verdünnungsmittels bei Temperaturen zwischen −70 und +150° C umsetzt;

5) ein Verfahren zur Herstellung von 1-Aryl-4-methylpenta-1,3-dienen der Formel II (oben), worin

$R^1$, $R^2$ und $R^3$ die oben angegebene Bedeutung haben,

das dadurch gekennzeichnet ist, dass man β-Arylacroleine der Formel VII

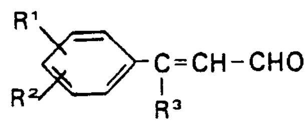 (VII)

in welcher

$R^1$, $R^2$ und $R^3$ die oben angegebene Bedeutung haben,

mit Triphenylisopropylphosphoran der Formel VIII

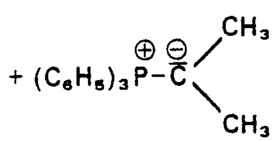 (VIII)

gegebenfalls unter Verwendung von Verdünnungsmitteln bei Temperaturen zwischen −70 und +50° C umsetzt;

6) ein Verfahren zur Herstellung von 1-Aryl-4-methylpenta-1,3-dienen der Formel II (oben), worin

$R^1$, $R^2$ und $R^3$ die oben angegebene Bedeutung haben,

das dadurch gekennzeichnet ist, dass man β-Arylacroleine der Formel VII (oben), worin

$R^1$, $R^2$ und $R^3$ die oben angegebene Bedeutung haben, mit Isopropylmagnesiumchlorid der Formel IX

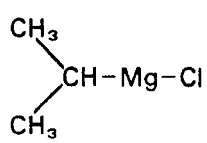 (IX)

gegebenenfalls in Gegenwart von Verdünnungsmitteln bei Temperaturen zwischen −70 und +50° C umsetzt, und anschliessend die dabei gebildeten Produkte durch Behandeln mit Säuren bei 0 bis 50° C dehydratisiert;

7) ein Verfahren zur Herstellung von β-Arylacroleinen der Formel VII (oben), worin

$R^1$, $R^2$ die oben angegebene Bedeutung hat, und

$R^3$ für Halogen steht,

dadurch gekennzeichnet, dass man Arylmethylketone der Formel X

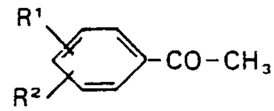 (X)

in welcher $R^1$ und $R^2$ die oben angegebene Bedeutung haben, mit Dimethylformamid und Phosphoroxyhalogenid bei Temperaturen zwischen 0 und 100° C ohne Zusatz eines weiteren Verdünnungsmittels umsetzt und die Reaktionsprodukte ohne destillative Aufarbeitung isoliert.

Überraschenderweise können nach dem neuen Verfahren, welches mit geringerem Aufwand als das bekannte Verfahren durchzuführen ist, und bei dem einfach herzustellende Ausgangsverbindungen eingesetzt werden können, 3-(2-Arylvinyl)-2,2-dimethylcyclopropan-1-carbonsäureester in guten Ausbeuten hergestellt werden.

Nach dem erfindungsgemässen Verfahren 1 werden vorzugsweise 3-(2-Chloro-2-arylvinyl)-2,2-dimethylcyclopropan-1-carbonsäureester der Formel I hergestellt,

in welcher

$R^1$ für $C_1$ bis $C_4$-Alkoxy, $C_1$ bis $C_4$-Alkylthio, $C_1$ bis $C_2$-Fluoralkoxy, $C_1$ bis $C_2$-Chlorfluoralkoxy oder $C_1$ bis $C_2$-Fluoralkylthio steht,

$R_2$ für Wasserstoff, Methoxy oder zusammen mit $R^1$ für $C_1$ bis $C_2$-Alkylendioxy oder $C_1$ bis $C_2$-Fluoralkylendioxy steht,

$R^3$ für Chlor steht, und

R für den Rest eines gegebenenfalls substituierten Phenoxybenzylalkohols der allgemeinen Formel XI

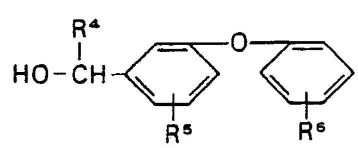 (XI)

steht, worin

$R^4$ für Wasserstoff, Cyano oder Äthinyl steht, und

$R^5$ und $R^6$ für Wasserstoff oder Fluor stehen.

Die allgemeinen Formel (I) umfasst die verschiedenen Isomeren und deren Mischungen.

Verwendet man beim erfindungsgemässen Verfahren 1 als Ausgangsstoffe beispielsweise 1-Chloro-1-(4'-methoxyphenyl)-4-methylpenta-1,3-dien und Diazoessigsäureäthylester, so kann die Reaktion dieser Verbindungen durch folgendes Formelschema skizziert werden:

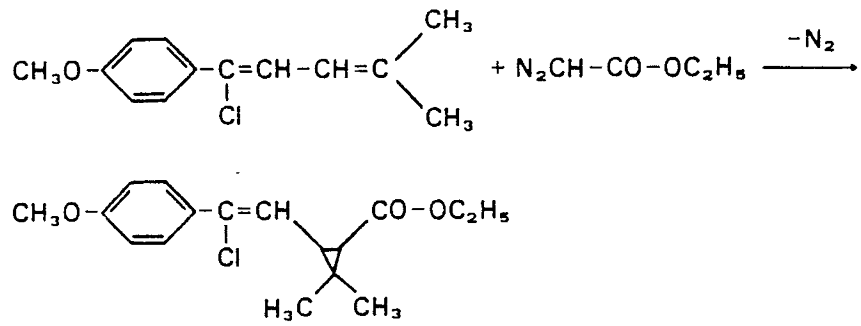


Die als Ausgangsstoffe für Verfahren 1 zu verwendenden Verbindungen sind durch die Formeln II und III definiert. Vorzugsweise stehen darin $R^1$, $R^2$ und $R^3$ für diejenigen Reste, welche bei der Definition der Reste $R^1$, $R^2$ und $R^3$ in Formel I als bevorzugt genannt wurden. Die Diazoessigsäureester der Formel III sind literaturbekannte Verbindungen oder können analog zu bekannten Verfahren hergestellt werden. Als Beispiele seien genannt: Diazoessigsäuremethylester und -äthylester.

Die neue 1-Aryl-4-methylpenta-1,3-diene der Formel II können nach den unter 3, 4, 5 und 6 angegebenen Verfahren hergestellt werden. Als Beispiele für die Verbindungen der Formel II seien genannt:

1-(4-Methoxyphenyl)-, 1-(4-Methylthiophenyl)-, 1-(4-Trifluoromethoxyphenyl)-, 1-(4-Trifluoromethylthiophenyl)-, 1-(3,4-Methylendioxyphenyl)-, 1-(3,4-Difluoromethylendioxyphenyl)- und 1-(3,4-Trifluoroäthylendioxyphenyl)-1-chloro-4-methylpenta-1,3-dien.

Verwendet man bei Verfahren 3 als Ausgangsstoffe beispielsweise ein Gemisch aus 2-Methyl-5-oxo-5-(4-methoxyphenyl)-2-penten und 2-Methyl-5-oxo-5-(4-methoxyphenyl)-3-penten, so kann die Reaktion dieser Verbindungen mit Phosphorpentachlorid, welcher eine Chlorwasserstoffeliminierung folgt, durch nachstehendes Formelschema skizziert werden:

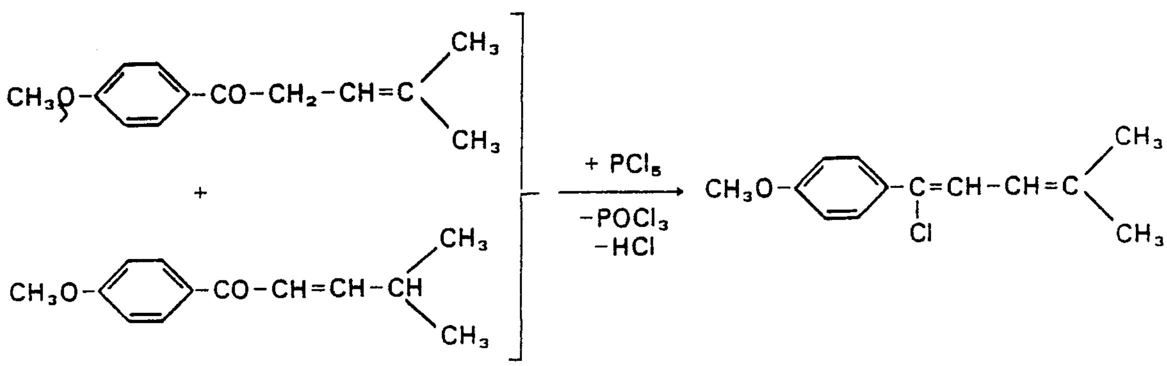


Die bei Verfahren 3 zu verwendenden Ausgangsstoffe sind durch die Formeln IVa und IVb definiert. Die bevorzugten und besonders bevorzugten Reste $R^1$ und $R^2$ sind die gleichen, wie bei den nach Verfahren 1 herzustellenden Verbindungen der Formel I.

Die Ausgangsverbindungen der Formeln IVa und IVb sind teilweise bekannt und/oder können nach einem bekannten Verfahren hergestellt werden [vgl. „J. Org. Chem." *25* (1960), 272]. Man erhält die Verbindungen der Formeln IVa und IVb beispielsweise, indem man Arylmethylketone der Formel X (oben) mit Isobutyraldehyd umsetzt, und die Umsetzungsprodukte in Gegenwart von Natriumacetat destilliert.

Als Beispiele für die Arylmethylketone der Formel X seien genannt:

4-Methoxyacetophenon und 3,4-Methylendioxyacetophenon.

Zur Durchführung von Verfahren 3 werden die Ausgangsstoffe der Formel IVa bzw. IVb bzw. deren Gemische in einem Verdünnungsmittel vorgelegt und mit Phosphorpentahalogenid, bevorzugt Phosphorpentachlorid, versetzt. Im Gegensatz zur allgemein üblichen Arbeitsweise bei der Umsetzung von Ketonen mit Phosphorpentachlorid (vgl. Houben-Weyl, „Methoden der organischen Chemie", Bd. 5/3, S. 912) wird Verfahren 3 vorzugsweise in Gegenwart eines Verdünnungsmittels durchgeführt. Als Verdünnungsmittel kommen beispielsweise Kohlenwasserstoffe, wie Benzin, Petroläther, Pentan, Hexan, Cyclohexan, Benzol, Toluol, Xylol oder Chlorkohlenwasserstoffe, wie Methylenchlorid, Tetrachlorkohlenstoff, Dichloräthan oder Chlorbenzol sowie Nitrile wie Acetonitril in Frage. Vorzugsweise werden Toluol und/oder Cyclohexan als Verdünnungsmittel verwendet. Die Umsetzung ungesättigter Ketone mit Phosphorpentachlorid ist praktisch immer problematisch und gibt Anlass zu einer Vielzahl von Nebenreaktionen. So kann beispielsweise eine Chlorierung in Allylstellung bzw. in $\alpha$-Stellung zur Carbonylgruppe erfolgen. Der hierbei wie auch bei der Eliminierung entstehende Chlorwasserstoff kann

sich wieder an eine Doppelbindung addieren. Ausserdem können auch Doppelbindungen durch Phosphorpentachlorid chloriert werden.

Es wurde gefunden, dass sich diese Nebenreaktionen praktisch vollständig vermeiden lassen, wenn man in Gegenwart von Verdünnungsmitteln und bei relativ tiefer Temperatur arbeitet und anschliessend bei der gleichen oder bei einer niedrigeren Temperatur eine Base zusetzt, sodass das Vorhandensein von Chlorwasserstoff bei höheren Temperaturen vermieden wird. Das Verfahren wird im allgemeinen bei Temperaturen zwischen −40 und +30° C, vorzugsweise zwischen −10 und +20° C durchgeführt.

Als Säurebindemittel können bei Verfahren 3 praktisch alle technisch gebräuchlichen Basen verwendet werden. Hierzu gehören Hydroxide, wie z.B. Natrium- und Kaliumhydroxid, Carbonate, wie z.B. Natrium- und Kaliumcarbonat sowie Alkoholate, wie z.B. Natriummethylat und -äthylat. Vorzugsweise werden wässrige Lösungen von Alkalihydroxiden oder -carbonaten in Gegenwart von Phasentransferkatalysatoren eingesetzt. Es können die üblichen Phasentransferkatalysatoren verwendet werden; vorzugsweise werden Tetraalkyl- oder Aralkyltrialkylammoniumsalze, wie z.B. Benzyltriäthylammoniumchlorid oder Tetrabutylammoniumbromid eingesetzt.

Die nach Verfahren 3 hergestellten 1-Aryl-4-methylpenta-1,3-diene können im allgemeinen durch Destillation gereinigt werden. Soweit diese Diene nicht unzersetzt destillierbar sind, erfolgt die Reinigung durch sogenanntes Andestillieren, d.h. durch längeres Erhitzen auf mässig erhöhte Temperaturen unter vermindertem Druck.

Verwendet man bei Verfahren 4 als Ausgangsstoffe beispielsweise 4-Trifluoromethylthiophenyl-α-chlorobenzylphosphonsäurediäthylester und Dimethylacrolein, so kann die Reaktion dieser Verbindungen durch folgendes Formelschema skizziert werden:

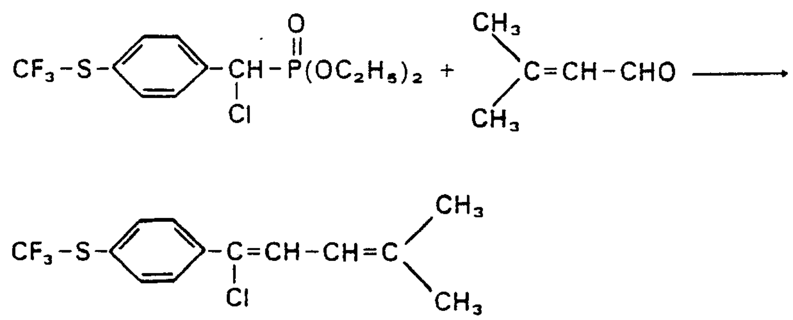

Die bei Verfahren 4 zu verwendenden Ausgangsstoffe sind durch die Formeln V und VI definiert. Die bevorzugten Reste $R^1$, $R^2$ und $R^4$ sind die gleichen wie bei den nach Verfahren 1 herzustellenden Verbindungen der Formel I. Dimethylacrolein VI ist eine literaturbekannte Verbindung.

Die α-Chloroarylmethanphosphonsäureester der Formel V sind teilweise bekannt. Sie können durch Umsetzung von α-Hydroxyarylmethanphosphonsäureestern der Formel XI

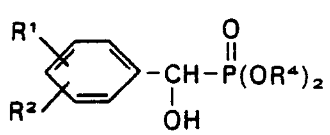 (XI)

in welcher

$R^1$, $R^2$ und $R^4$ die oben angegebene Bedeutung haben, mit einem Chlorierungsmittel wie z.B. Thionylchlorid bei Temperaturen zwischen 0 und 100° C, vorzugsweise bei 20 bis 80° C, hergestellt werden [vgl. „Chimia" *28* (1974), S. 656-657; „J. Am. Chem. Soc." *87* (1965), S. 2777-2778].

Die α-Hydroxyarylmethanphosphonsäureester der Formel XI sind teilweise bekannt. Man erhält sie nach bekannten Verfahren, im allgemeinen durch Umsetzung von Aldehyden der Formel XII

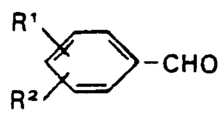

in welcher

$R^1$ und $R^2$ die oben angebene Bedeutung haben, mit Phosphorigsäureestern der Formel XIII

$$H-\overset{O}{\overset{\|}{P}}(OR^4)_2 \qquad (XIII)$$

in welcher $R^4$ die oben angegebene Bedeutung hat, gegebenenfalls in Gegenwart eines Katalysators wie z.B. Triäthylamin bei Temperaturen zwischen 0 und 150° C, vorzugsweise bei 20 bis 100° C [vgl. Houben-Weyl, „Methode der organischen Chemie", 4. Aufl. (1963), Bd. 12/1, S. 475-483].

Als Beispiele für die Aldehyde der Formel XII seien genannt:

4-Methoxybenzaldehyd, 4-Trifluormethoxybenzaldehyd, 4-Trifluoromethylthiobenzaldehyd, 3,4-Methylendioxybenzaldehyd (Piperonal), 3,4-Difluoromethylendioxybenzaldehyd und 4-Trifluoromethylbenzaldehyd.

Als Beispiele für die Phosphorigsäureester der Formel XIII seien Phosphorigsäuredimethylester und Phosphorigsäurediäthylester genannt:

Verfahren 4 ist eine Variante der Wittig-Horner-Reaktion. Aus der Literatur sind für diese Variante nur wenige Beispiele bekannt. Als Base wird nach dem Stand der Technik Natriumhydrid verwendet, welches ein teures und schwierig zu handhabendes Reagenz ist. Dagegen kann man nach dem neuen Verfahren wesentlich einfacher und kosten-

günstiger arbeiten. Man kann die Umsetzung bei Raumtemperatur in wasserhaltigen Reaktionsmedien durchführen und an Stelle von Natriumhydrid billigere Basen verwenden.

Verfahren 4 wird vorzugsweise unter verwendung von Verdünnungsmitteln durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan. Alkohole wie Methanol, Äthanol, n- und iso-Propanol, n-, iso-, sek.- und tert.-Butanol sowie Dimethylsulfoxid.

Bei Arbeiten im 2 phasen-Medium werden neben 50%iger wässriger Natronlauge mit Wasser praktisch nicht mischbare Solventien wie z.B. Benzin, Benzol oder Toluol verwendet.

Als Basen können die bei Carbonylolefinierungen üblichen Basen verwendet werden. Genannt seien Alkalihydroxide wie z.B. Natrium- und Kaliumhydroxid, Alkalialkoholate wie z.B. Natrium- und Kaliummethylat, -äthylat, -n- und -iso-propylat, -n-, -iso-, -sek.- und tert.- butylat, Alkalihydride wie z.B. Natriumhydrid sowie Alkyllithiumverbindungen wie z.B. Butyllithium. Vorzugsweise werden als Basen Natrium- und Kaliumhydroxid sowie Natriummethylat und -äthylat verwendet.

Die Reaktionstemperaturen liegen im allgemeinen zwischen −70 und +150° C, vorzugsweise zwischen −10 und +50° C. Die Umsetzung wird im allgemeinen bei Normaldruck durchgeführt. Zur Durchführung von Verfahren 4 werden die Komponenten gewöhnlich in äquimolaren Mengen eingesetzt. Lediglich die Basen werden gewöhnlich in grösserem Überschuss eingesetzt; bei Arbeiten im 1phasigen System bis zu 30 mol%, vorzugsweise bis zu 15 mol%; bei Verwendung von 50%iger Natronlauge als zweiter Phase im allgemeinen das 5 bis 15fache der stöchiometrisch erforderlichen Menge.

Alkoholate werden gegebenenfalls *in situ* aus den Alkoholen und Alkalimetallen hergestellt.

Im allgemeinen wird die Base und gegebenenfalls der Katalysator in einem oder mehreren der genannten Verdünnungsmittel vorgelegt. Dazu werden, gegebenenfalls in einem der angegebenen Solventien gelöst, die Reaktionskomponenten α-Chlorobenzylphosphonsäureester V und Dimethylacrolein VI — in der angegebenen Reihenfolge zugegeben. Zur Vervollständigung der Reaktion wird die Mischung unter Rühren mehrere Stunden im angegebenen Temperaturbereich gehalten. Zur Aufarbeitung versetzt man die Reaktionsmischung mit Wasser, säuert gegebenenfalls mit Salzsäure an und extrahiert mit Methylenchlorid. Die organische Phase wird getrocknet und das Lösungsmittel wird im Vakuum andestilliert. Das als Rückstand verbleibende Produkt wird gegebenenfalls durch Vakuumdestillation, oder falls es nicht unzersetzt destillierbar ist, durch sogenanntes Andestillieren, d.h. durch längeres Erhitzen unter vermindertem Druck auf mässig erhöhte Temperaturen, gereinigt. Zur Charakterisierung dient der Siedepunkt oder der Brechungsindex.

Verwendet man bei Verfahren 5 als Ausgangsstoffe β-Chloro-β-(3,4-methylendioxyphenyl)-acrolein und Isopropylphosphoran, so kann die Reaktion dieser Verbindungen durch folgendes Formelschema skizziert werden:

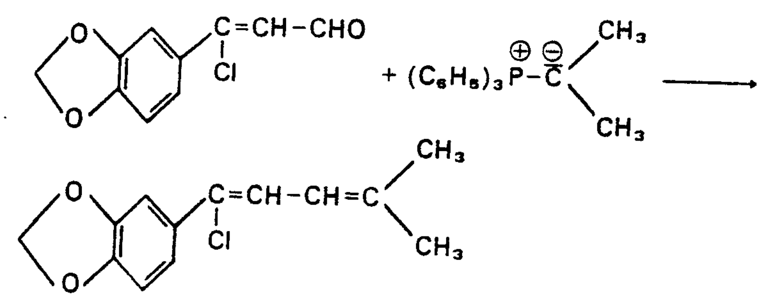

Die Herstellung des Phosphorans VIII ist bekannt.

Die Acroleinderivate der Formel VII sind teilweise bekannt. Ein neues Verfahren 7 zu ihrer Herstellung wird weiter unten beschrieben.

Die bei Verfahren 5 zu verwendenden Ausgangsstoffe sind durch die Formeln VII und VIII definiert. Die bevorzugten und besonders bevorzugten Reste und Substituenten sind die gleichen wie bei den nach Verfahren 1 herzustellenden Verbindungen der Formel I. Als Beispiele für die Acroleinderivate der Formel VII seien genannt:

β-(4-Methoxyphenyl)- und β-(3,4-Methylendioxyphenyl)acrolein und die entsprechenden β-Chloroacroleine.

Verfahren 5 wird nach den bei Carbonylolefinierungen nach Wittigs üblichen Methoden durchgeführt.

Verwendet man bei Verfahren 6 als Ausgangsstoffe β-Chloro-β-(4-methoxyphenyl)acrolein und Isopropylmagnesiumchlorid, so kann die Reaktion dieser Verbindungen durch folgendes Formelschema skizziert werden:

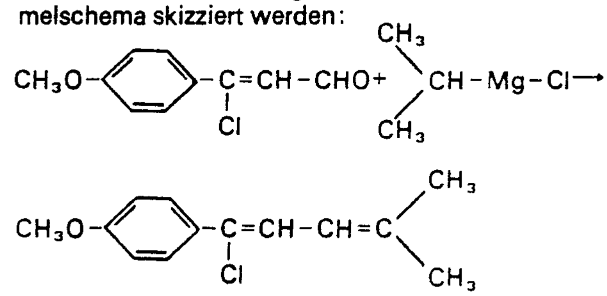

Isopropylmagnesiumchlorid kann nach einem bekannten Verfahren hergestellt werden [vgl. „Chem. Ber." *69* (1936), 1766].

Die bei Verfahren 6 zu verwendenden Ausgangsstoffe sind durch die Formeln VII und IX definiert. Die bevorzugten und besonders bevorzugten Reste und Substituenten sind die gleichen wie bei den nach Verfahren 1 herzustellenden Verbindungen der Formel I. Als Beispiele für die Acroleinderivate der Formel VII seien genannt:

β-(4-Methoxyphenyl)- und β-(3,4-Methylendioxyphenyl)acrolein und die entsprechenden β-Chloroacroleine.

Verfahren 6 ist eine Grignard-Reaktion mit nachfolgender Dehydratisierung. Die Grignard-Reaktion kann auf bekannte Weise durchgeführt werden. Es ist als überraschend anzusehen, dass aus dem intermediär gebildeten sekundären Alkohol verhältnismässig leicht Wasser eliminiert werden kann. Die Dehydratisierung erfolgt mit Säuren, vorzugsweise mit Schwefelsäure bei Temperaturen zwischen 0 und 50° C, vorzugsweise bei 20 bis 45° C. Die Aufarbeitung erfolgt nach Eingiessen in Wasser durch Extraktion nach üblichen Methoden.

Verwendet man bei Verfahren 7 als Ausgangsstoffe beispielsweise 4-Methoxyacetophenon, Dimethylformamid und Phosphoroxychlorid, so kann die Reaktion durch folgendes Formelschema skizziert werden:

Die bei Verfahren 7 als Ausgangsstoffe zu verwendenden Arylmethylketone sind durch Formel X definiert. Die bevorzugten und besonders bevorzugten Reste und Substituenten sind die gleichen wie bei den nach Verfahren 7 herzustellenden Verbindungen der Formel I. Als Beispiele für die Arylmethylketone der Formel seien genannt:

4-Methoxyacetophenon, 3,4-Methylendioxyacetophenon.

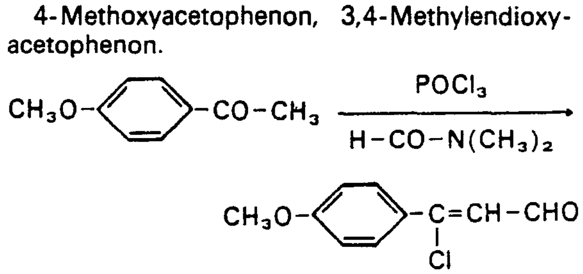


Man erhält nach Verfahren 7 β-Chloro-β-arylacroleine der Formel VII in besonders einfacher Weise und mit verbesserter Ausbeute und Qualität, wenn man auf den in der Literatur [„Proc. Chem. Soc." (London) 1958, 227; „Angew. Chem." *71* (1959), 573; „Chem. Ber." *93* (1960), 2746 und „Chem. Ber." *98* (1965), 3554] empfohlenen Zusatz eines Verdünnungsmittels und die verlustreiche destillative Aufarbeitung verzichtet und die Produkte nach schonender Hydrolyse bei tiefer Temperatur in kristalliner Form isoliert. Man geht bei Verfahren 7 so vor, dass man das Keton der Formel X in Dimethylformamid löst, und bei Temperaturen zwischen 0 und 100° C, vorzugsweise zwischen 20 und 60° C, mit Phosphoroxychlorid versetzt und bei der gleichen Temperatur 1 bis 5 h nachrührt. Anschliessend wird auf Eiswasser gegeben und mit Natronlauge ein pH-Wert von ungefähr 5 eingestellt. Die Temperatur soll dabei nicht über 25° C steigen. Nach einigem Nachrühren wird abgesaugt, mit Wasser gewaschen und im Vakuum getrocknet.

Das erfindungsgemässe Verfahren 1 wird in Gegenwart von Katalysatoren durchgeführt. Als solche kommen Kupfer und Kupferverbindungen verschiedener Oxidationsstufen — auch Gemische — in Frage, wie z.B. Kupfer (als Pulver oder Bronze), Kupfer(I)- und -(II)chlorid, Kupfer(I)- und -(II)oxid sowie Kupfer(II)sulfat. Die Reaktionstemperatur kann innerhalb eines grösseren Bereichs variiert werden. Im allgemeinen arbeitet man zwischen 50 und 200° C, vorzugsweise bei 80 bis 150° C.

Das erfindungsgemässe Verfahren wird im allgemeinen bei Normaldruck durchgeführt. Bei der Durchführung von Verfahren 1 kann das Mengenverhältnis der Ausgangsverbindungen in einem grösseren Bereich variiert werden, allgemein arbeitet man bei einem Mengenverhältnis Diazoessigsäureester zu α-Aryl-4-methylpenta-1,3-dien zwischen 1 : 1 und 1 : 20. Im allgemeinen werden etwa ⅔ der einzusetzenden Menge an 1-Chloro-1-aryl-4-methylpenta-1,3-dien (II) zusammen mit dem Katalysator auf die erforderliche Reaktionstemperatur erhitzt und dann tropfenweise mit dem Diazoessigsäureester (III) versetzt, welcher mit dem restlichen Drittel des Diens (II) verdünnt ist. Nach Ende der Stickstoffentwicklung wird das Reaktionsgemisch abgekühlt, mit einem mit Wasser nicht mischbaren Lösungsmittel, wie z.B. Methylenchlorid, verdünnt und filtriert. Das Filtrat wird mit Wasser gewaschen, getrocknet und destillativ aufgearbeitet. Zur Charakterisierung der Produkte dienen die Siedepunkte.

Nach dem erfindungsgemässen Verfahren herstellbare Verbindungen der Formel I können als Zwischenprodukte zur Herstellung von Insektiziden verwendet werden (vgl. DE-OS Nr. 2738150). Diese Insektizide sind Gegenstand der europäischen Anmeldung Nr. 80.102002.5.

*Beispiel*

*Phaedon*-Larven-Test

Lösungsmittel: 3 Gewichsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter *(Brassica oleracea)* werden durch Tauchen in die Wirkstoffzubereitung der gewünschten Konzentration behandelt und mit Meerrettichblattkäfer-Larven *(Phaedon cochleariae)* besetzt, solange die Blätter noch feucht sind.

Nach der gewünschten Zeit wird die Abtötung in Prozent bestimmt. Dabei bedeutet 100%, dass

alle Käferlarven abgetötet wurden; 0% bedeutet, dass keine Käferlarven abgetötet wurden.

Bei diesem Test zeigt z.B. die folgende Verbindung der Herstellungsbeispiele überlegene Wirksamkeit gegenüber dem Stand der Technik:

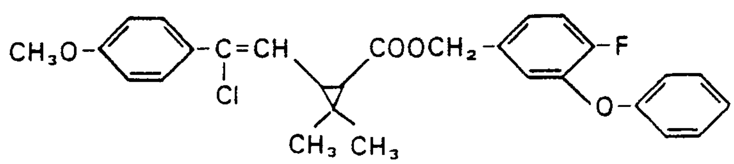

*Herstellungsbeispiele*

Beispiele zu Verfahren 1:

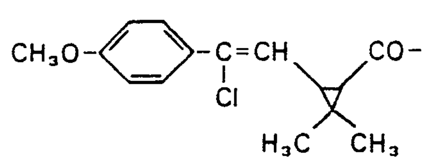

Eine Mischung von 44,4 g (0,2 mol) 1-(4-Methoxyphenyl)-1-chloro-4-methylpenta-1,3-dien, 1,2 g Kupferpulver und 1,5 g Kupfersulfat (wasserfrei) wird auf 110° C erhitzt, und dann unter Rühren ebenfalls bei 110° C eine Mischung von 22,2 g (0,1 mol) 1-(4-Methoxyphenyl)-1-chloro-4-methylpenta-1,3-dien und 34,2 g (0,3 mol) Diazoessigsäureäthylester, sehr langsam, innerhalb von 5 h, zugetropft. Nach beendeter Stickstoffentwicklung wird die Mischung abgekühlt, mit 500 ml Methylenchlorid verdünnt und dann filtriert. Das Filtrat wird mit 500 ml Wasser ausgeschüttelt, anschliessend wird die organische Phase abgetrennt, über Magnesiumsulfat getrocknet und dann das Lösungsmittel im Wasserstrahlvakuum abdestilliert. Der ölige Rückstand wird im Vakuum destilliert. Man erhält dabei zwei Fraktionen:

Fraktion 1: Siedepunkt 124-145° C/2 Torr

Fraktion 2: Siedepunkt 155-175° C/2 Torr

Fraktion 1 erweist sich als nicht umgesetztes 1-(4-Methoxyphenyl)-1-chloro-4-methylpenta-1,3-dien. Fraktion 2 wird nochmals destilliert. Man erhält dann 11,2 g 2,2-Dimethyl-3-[2-chloro-2-(4-methoxyphenyl)vinyl]cyclopropancarbonsäureäthylester (Isomerengemisch) als gelbes Öl mit dem Siedepunkt 160-172° C/2 Torr.

Beispiel zu Verfahren 3:

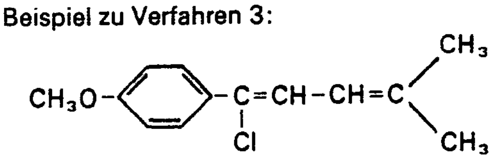

Man löst 17 g Kaliumhydroxid in 125 ml Wasser und 125 ml Methanol und gibt 150 g 4-Methoxyacetophenon hinzu. Bei 50° C werden 80 g Isobutyraldehyd innerhalb von 1,5 h zugetropft. Nach 3,5 h wird auf Raumtemperatur abgekühlt und mit ca. 20 ml Essigsäure neutralisiert. Das dimere Kondensationsprodukt wird abgesaugt, mit Methanol gewaschen und an der Luft getrocknet. Ausbeute 175 g. Der Feststoff wird mit 5 g Natriumacetat vermischt und im Wasserstrahlvakuum destilliert. Man erhält 165 g eines bei Raumtemperatur erstarrenden gelben Öls, das ein Gemisch aus 2-Methyl-5-oxo-5-(4-methoxyphenyl)-2-penten und 2-Methyl-5-oxo-5-(4-methoxyphenyl)-3-penten darstellt. Siedepunkt des Isomerengemisches: 158-165° C/17 mbar.

Man löst 102 g (0,5 mol) des oben erhaltenen Isomerengemisches in 500 ml Toluol und dosiert 104 g Phosphorpentachlorid bei 0° C langsam hinzu. Man rührt solange bei 0-10° C, bis alles $PCl_5$ in Lösung gegangen ist, gibt 5 g Tetrabutylammoniumbromid zu und tropft unter guter Kühlung bei 0-10° C 224 g (2 mol) 50%ige Kalilauge zu. Anschliessend trennt man die Toluolphase sofort ab, wäscht neutral und rotiert ein. Anschliessend wird das rohe Dien überdestilliert (Siedepunkt 125-165° C/0,8 mm) und anschliessend durch eine zweite Destillation gereinigt. Man erhöt 75 g 1-(4-Methoxyphenyl)-1-chloro-4-methylpenta-1,3-dien vom Siedepunkt 112-119° C/0,2 mm.

Beispiel zu Verfahren 4:

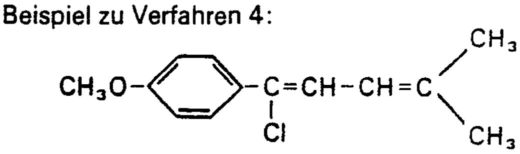

4,6 g (0,2 mol) Natrium werden portionsweise in 100 ml trockenem Äthanol gelöst. Wenn sich alles Natrium aufgelöst hat, werden 100 ml wasserfreies Tetrahydrofuran zugesetzt und bei 0° C unter Rühren 58,4 g (0,2 mol) 4-Methoxy-α-chlorobenzylphosphonsäurediäthylester, gelöst in 50 ml wasserfreiem Tetrahydrofuran, zugetropft. Nachdem noch 1 h bei 0-10° C nachgerührt wurde, werden 16,8 g (0,2 mol) β,β-Dimethylacrolein, in 30 ml wasserfreiem Tetrahydrofuran gelöst, unter Rühren zugetropft. Anschliessend wird noch 12 h bei Raumtemperatur nachgerührt. Die Reaktionsmischung wird dann mit 600 ml Wasser versetzt und zweimal mit je 300 ml Methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 25 g 1-(4-Methoxyphenyl)-1-chloro-4-methylpenta-1,3-dien (Isomerengemisch) als gelbes, nach einiger Zeit teilweise kristallisierendes Öl, mit dem Siedepunkt 130-145° C/2 Torr.

Analog erhält man

1-(3,4-Methylendioxyphenyl)-1-chloro-4-methylpenta-1,3-dien

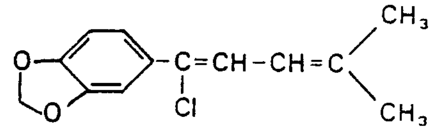

Die als Vorprodukte benötigten α-Hydroxybenzylphosphonsäureester der Formel

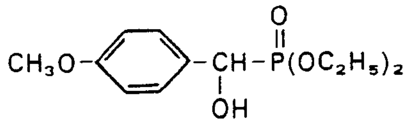

können wie folgt hergestellt werden:

In ein Gemisch aus 20,7 g (0,15 mol) Diäthylphosphit und 1,09 g (0,0109 mol) Triäthylamin werden innerhalb 1 h unter Wasserkühlung bei 50-70° C 20,4 g (0,150 mol) p-Methoxybenzaldehyd eingeleitet. Der Reaktionsansatz wird 1 h bei 70° C nachgerührt. Nach dem Abkühlen wird der Ansatz mit 40 g Toluol aufgenommen und mehrfach mit verdünnter Salzsäure und kaltem Wasser nachgewaschen. Die organische Schicht wird abgetrennt und im Vakuum vom Lösungsmittel befreit. Die Ausbeute beträgt 37 g 4-Methoxy-α-hydroxybenzylphosphonsäurediäthylester.

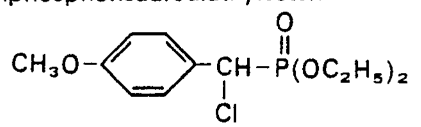

Einem Gemisch aus 20 g (0,073 mol) 4-Methoxy-α-hydroxybenzylphosphonsäurediäthylester, 65 g Dichloromethan und 5,8 g (0,0725 mol) Pyridin werden bei 20-40° C unter leichter Wasserkühlung innerhalb von etwa 1 h 9,2 g (0,0768 mol) Thionylchlorid hinzugesetzt. Das Reaktionsgemisch wird dann 3 h unter Rückfluss erhitzt und 12 h ohne weitere Wärmeeinwirkung nachgerührt. Das Gemisch wird auf etwa 100 g Eiswasser gegossen, die organische Phase abgetrennt und getrocknet. Nach Abdestillieren des Lösungsmittels wird der Rückstand bei 6 Torr und 45° C eingeengt. Man erhält 21 g 4-Methoxy-α-chlorobenzylphosphonsäurediäthylester als gelbes viskoses Öl mit einer Reinheit von 98,6% (Gaschromatogramm) und einem Brechungsindex von $n_D^{24}$ : 1,5188.

Beispiel zu Verfahren 5:

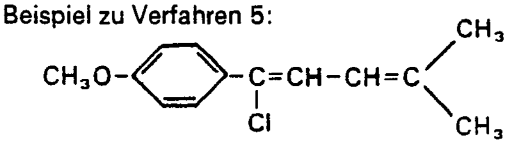

Zu einer Suspension von 22,7 g (0,0525 mol) Triphenylisopropylphosphoniumjodid in 100 ml Tetrahydrofuran werden bei 0° C 0,055 mol n-Butyllithium in 30 ml Hexan getropft. Es wird 1 h bei 0° C nachgerührt. Dann wird in einem Guss eine Lösung von 10 g (0,05 mol) β-(4-Methoxyphenyl)-β-chloroacrolein in 100 ml Tetrahydrofuran zugegeben und dann 10 h bei 20° C nachgerührt. Das Reaktionsgemisch wird darauf eingeengt, mit 100 ml Wasser versetzt und zweimal mit je 50 ml Äther extrahiert. Die vereinigten Ätherextrakte werden über Natriumsulfat getrocknet und eingeengt. Es verbleibt ein Rückstand, der bei Zugabe von ca. 50 ml Petroläther kristallisiert. Die Kristalle werden abgesaugt und noch einmal aus Petroläther umkristallisiert.

Man erhält 8 g 1-(4-Methoxyphenyl)-1-chloro-4-methylpenta-1,3-dien in Form von hellgelben Kristallen.

Beispiel zu Verfahren 6:

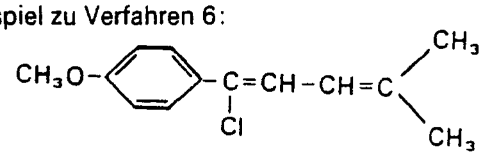

Zu einer Lösung von 0,4 mol Isopropylmagnesiumchlorid in 40 ml Isopropylchlorid [hergestellt nach J. Houben *et al.* „Chem. Ber." *69* (1936), 1766] werden bei 20° C 39,3 g (0,2 mol) β-Chlor-β-(4-methoxyphenyl)acrolein in 200 ml Tetrahydrofuran getropft. Es wird 12 h nachgerührt. Dann wird die Reaktionsmischung unter Rühren in eine Mischung von 500 g Eis und 100 ml Schwefelsäure gegossen. Es wird zweimal mit je 100 ml Äther extrahiert. Die vereinigten Ätherextrakte werden über Natriumsulfat getrocknet und bei 50° C am Rotationsverdampfer eingeengt. Der Rückstand wird in 50 ml Eisessig aufgenommen. Zu dieser Lösung werden 2 ml konzentrierter Schwefelsäure in der Weise getropft, dass die Temperatur 45° C nicht übersteigt. Es wird 4 h bei 20° C nachgerührt. Dann wird die Reaktionsmischung in Wasser gegeben. Es wird zweimal mit je 100 ml Äther extrahiert. Die vereinigten Ätherextrakte werden über Natriumsulfat getrocknet, eingeengt und destilliert. Man erhält 22 g 1-(4-Methoxyphenyl)-1-chloro-4-methylpenta-1,3-dien in Form eines gelben Öles, das beim Abkühlen teilweise kristallisiert. Siedepunkt 140-145° C/ 4 mm.

Beispiel zu Verfahren 7:

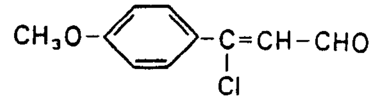

750 g (5 mol) 4-Methoxyacetophenon werden in 2,5 l Dimethylformamid gelöst, bei 40-45° C unter leichter Kühlung innerhalb von 2 h tropfenweise mit 1570 g Phosphoroxychlorid versetzt, 1 h verrührt, auf 25 l Eiswasser ausgetragen und durch Zutropfen von ca. 2,1 l konzentrierter Natronlauge ein pH-Wert von 5 eingestellt. Die Temperatur wird während der Neutralisation durch Einwerfen von Eis unter 25° C gehalten. Anschliessend wird noch 1 h bei pH 5 und bei einer Temperatur unter 25° C nachgerührt. Der helle kristalline Niederschlag wird abgesaugt, gründlich mit Wasser gewaschen und bei 50° C im Vakuum getrocknet. Ausbeute 730 g.

*Beispiel für Wirkstoffe gemäss Formel I*

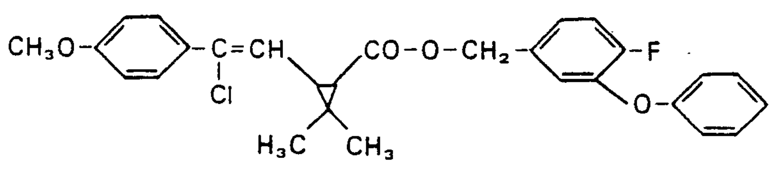

4,4 g (0,02 mol) 3-Phenoxy-4-fluorobenzylalkohol und 7,1 g (0,02 mol) 2,2-Dimethyl-3-[2-chloro-2(4-methoxyphenyl)vinyl]cyclopropancarbonsäurechlorid werden in 100 ml wasserfreiem Toluol gelöst und bei 20-25° C 2,5 g Pyridin, gelöst in 50 ml wasserfreiem Toluol, unter Rühren zugetropft. Anschliessend wird weitere 3 h bei 25-35° C gerührt. Das Reaktionsgemisch wird in 150 ml Wasser, dem 10 ml konzentrierte Salzsäure zugesetzt werden, gegossen, die organische Phase abgetrennt und nochmals mit 100 ml Wasser gewaschen. Anschliessend wird die Toluolphase über Natriumsulfat getrocknet und das Lösungsmittel im Wasserstrahlvakuum abdestilliert. Letzte Lösungsmittelreste werden durch kurzes Andestillieren bei 60° C/1 Torr Badtemperatur entfernt. Man erhält 8,1 g (84,5% der Theorie) 2,2-Dimethyl-3-[2-chloro-2-(4-trifluormethoxyphenyl)vinyl]cyclopropancarbonsäure-(4-fluoro-3-phenoxybenzyl)ester.

**Patentansprüche**

1. Verfahren zur Herstellung von Styrylcyclopropancarbonsäureestern der Formel I

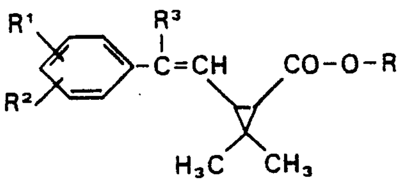

(I)

in welcher

R für $C_1$ bis $C_4$-Alkyl oder für einen bei Pyrethroiden üblichen Alkoholrest steht,

$R^1$ für Alkoxy oder Alkylthio steht, die gegebenenfalls durch Halogen substituiert sind,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht,

dadurch gekennzeichnet, dass man 1-Aryl-4-methylpenta-1,3-diene der Formel II

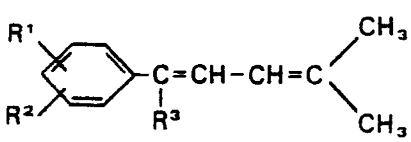

(II)

in welcher

$R^1$, $R^2$, $R^3$ die oben angegebene Bedeutung haben, mit Diazoessigsäureestern der Formel III

$$N_2CH-COO-R \quad (III)$$

in welcher

R die oben angegebene Bedeutung hat, in Gegenwart von Kupfer oder Kupferverbindungen bei Temperaturen zwischen 50 und 200° C umsetzt.

2. 1-Aryl-4-methylpenta-1,3-diene der Formel II

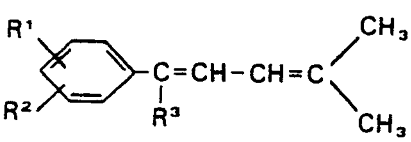

(II)

worin

$R^1$ für Alkoxy oder Alkylthio steht, die gegebenenfalls durch Halogen substituiert sind,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht.

3. Verfahren zur Herstellung von 1-Aryl-4-methylpenta-1,3-dienen der Formel II

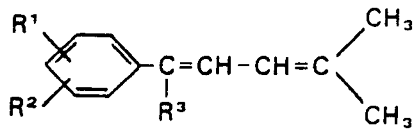

(II)

a) in welcher

$R^1$ für Alkoxy oder Alkylthio steht, die gegebenenfalls durch Halogen substituiert sind,

$R^2$ für Wasserstoff, Alkoxy oder gemeinsam mit $R^1$ für gegebenenfalls halogensubstituiertes Alkylendioxy steht, und

$R^3$ für Halogen steht,

das dadurch gekennzeichnet ist, dass man 3-Methylbuten-2-ylarylketone der Formel IVa

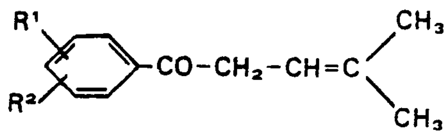

(IVa)

und/oder die dazu isomeren 3-Methylbuten-1-ylarylketone der Formel IVb

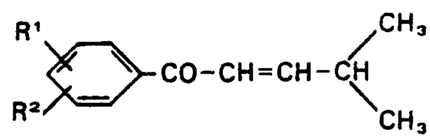

(IVb)

worin

$R^1$ und $R^2$ die oben angegebene Bedeutung haben, mit Phosphorpentahalogenid, gegebenenfalls unter Verwendung eines inerten Verdünnungsmittels, bei Temperaturen zwischen −40° C und +30° C umsetzt, und anschliessend das Reaktionsgemisch im gleichen Temperaturbereich mit einem Säurebindemittel, gegebenenfalls in Gegenwart eines Katalysators, behandelt, oder

b) in welcher

$R^1$ für Alkoxy oder Alkylthio steht, die gegebenenfalls durch Halogen substituiert sind,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht,

das dadurch gekennzeichnet ist, dass man Arylmethanphosphonsäureester der Formel V

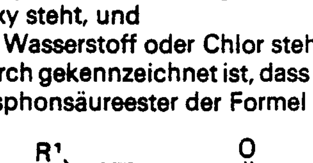

(V)

in welcher

$R^1$, $R^2$ und $R^3$ die oben angegebene Bedeutung haben und

$R^4$ für Alkyl oder Phenyl steht, oder worin die Reste $R^4$ zusammen für geradkettiges oder verzweigtes Alkandiyl stehen,

mit Dimethylacrolein der Formel VI

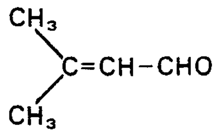

(VI)

in Gegenwart einer Base und gegebenenfalls unter Verwendung eines Verdünnungsmittels bei Temperaturen zwischen −70 und +150° C umsetzt, oder

c) worin

$R^1$ für Alkoxy oder Alkylthio steht, die gegebenfalls durch Halogen substituiert sind,

$R^2$ für Wassserstoff, Alkoxy oder gemeinsam mit $R^1$ für gegebenenfalls halogensubstituiertes Alkylendioxy steht, und

$R^3$ für Wasserstoff oder Chlor steht,

das dadurch gekennzeichnet ist, dass man β-Arylacroleine der Formel VII

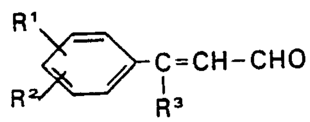

(VII)

in welcher

$R^1$, $R^2$ und $R^3$ die oben angegebene Bedeutung haben, mit Triphenylisopropylphosphoran der Formel VIII

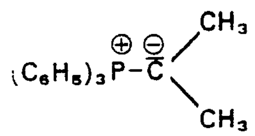

(VIII)

gegebenenfalls unter Verwendung von Verdünnungsmitteln bei Temperaturen zwischen −70 und +50° C umsetzt, oder

d) worin

$R^1$ für Alkoxy oder Alkylthio steht, die gegebenenfalls durch Halogen substituiert sind,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht,

das dadurch gekennzeichnet ist, dass man β-Arylacroleine der Formel VII (oben), worin $R^1$, $R^2$ und $R^3$ die oben angegebene Bedeutung haben, mit Isopropylmagnesiumchlorid der Formel IX

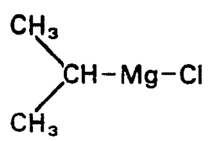

(IX)

gegebenenfalls in Gegenwart von Verdünnungsmitteln bei Temperaturen zwischen −70 und +50° C umsetzt, und anschliessend die dabei gebildeten, Produkte durch Behandeln mit Säuren bei 0 bis 50° C dehydratisiert.

**Claims**

1. Process for the preparation of styrylcyclopropanecarboxylic acid esters of the formula I:

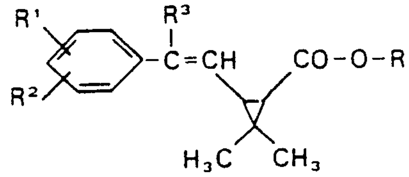

(I)

in which:

R représents $C_{1-4}$-alkyl or an alcohol radical customary in pyrethroids,

$R^1$ represents alkoxy or alkylthio, optionally substituted by halogen,

$R^2$ represents hydrogen or alkoxy or, together with $R^1$, represents optionally halogen-substituted alkylenedioxy, and

$R^3$ represents hydrogen or chlorine,

characterized in that 1-aryl-4-methylpenta-1,3-dienes of the formula II:

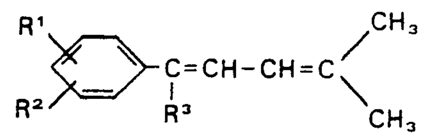

(II)

in which:

$R^1$, $R^2$ and $R^3$ have the meaning indicated above, are reacted with diazoacetic acid esters of the formula III:

$$N_2CH-COO-R \qquad (III)$$

in which:

R has the meaning indicated above, in the presence of copper or copper compounds at temperatures between 50 and 200° C.

2. 1-Aryl-4-methylpenta-1,3-dienes of the formula (II):

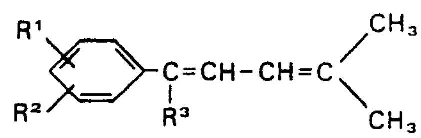

(II)

wherein:

$R^1$ represents alkoxy or alkylthio, optionally substituted by halogen,

$R^2$ represents hydrogen or alkoxy or, together with $R^1$, represents optionally halogen-substituted alkylenedioxy, and

$R^3$ represents hydrogen or chlorine.

3. Process for the preparation of 1-aryl-4-methyl-penta-1,3-dienes of the formula II:

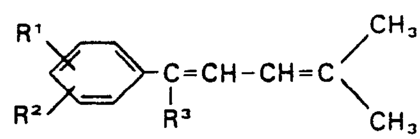

(II)

a) in which:

$R^1$ represents alkoxy or alkylthio, optionally substituted by halogen,

$R^2$ represents hydrogen or alkoxy or, together with $R^1$, represents optionally halogen-substituted alkylenedioxy, and

$R^3$ represents halogen,

characterized in that 3-methylbuten-2-ylaryl ketones of the formula IVa:

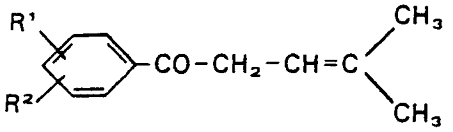

(IVa)

and/or the isomeric 3-methylbuten-1-ylaryl ketones of the formula IVb:

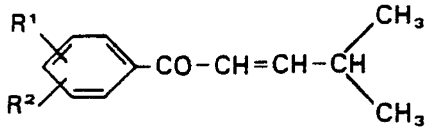

(IVb)

wherein:

$R^1$ and $R^2$ have the meaning indicated above, are reacted with phosphorus pentahalide, if appropriate using an inert diluent, at temperatures between −40° and +30° C, and the reaction mixture is then treated with an acid-binding agent in the same temperature range, if appropriate in the presence of a catalyst, or

b) in which:

$R^1$ represents alkoxy or alkylthio, optionally substituted by halogen,

$R^2$ represents hydrogen or alkoxy or, together with $R^1$, represents optionally halogen-substituted alkylenedioxy, and

$R^3$ represents hydrogen or chlorine,

characterized in that arylmethanephosphonic acid esters of the formula V:

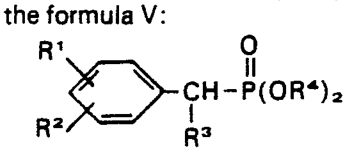

(V)

in which:

$R^1$, $R^2$ and $R^3$ have the meaning indicated above, and

$R^4$ represents alkyl or phenyl (or wherein the radicals $R^4$ together represent straight-chain or branched alkanediyl),

are reacted with dimethylacrolein of the formula VI:

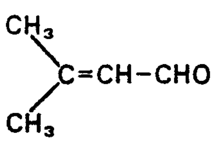

(VI)

in the presence of a base and if appropriate using a diluent, at temperatures between −70 and +150° C, or

c) wherein

$R^1$ represents alkoxy or alkylthio, optionally substituted by halogen,

$R^2$ represents hydrogen or alkoxy or, together with $R^1$, represents optionally halogen-substituted alkylenedioxy, and

$R^3$ represents hydrogen or chlorine,

characterized in that β-arylacroleins of the formula VII:

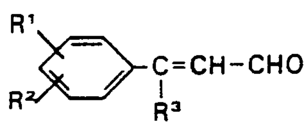

(VII)

in which:

$R^1$, $R^2$ and $R^3$ have the meaning indicated above, are reacted with triphenylisopropylphosphorane of the formula VIII:

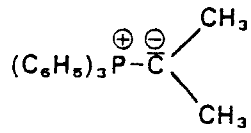

(VIII)

if appropriate using diluents, at temperatures between −70 and +50° C, or

d) wherein

$R^1$ represents alkoxy or alkylthio, optionally substituted by halogen,

$R^2$ represents hydrogen or alkoxy or, together with $R^1$, represents optionally halogen-substituted alkylenedioxy, and

$R^3$ represents hydrogen or chlorine,

which is characterized in that β-arylacroleins of the above formula VII, wherein $R^1$, $R^2$ and $R^3$ have the meaning indicated above, are reacted with isopropylmagnesium chloride of the formula IX:

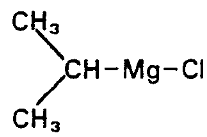

(IX)

if appropriate in the presence of diluents, at temperatures between −70 and +50° C and the reaction products thereby formed are then dehydrated by treatment with acids at 0 to 50° C.

## Revendications

1. Procédé de préparation d'esters d'acides styrylcyclopropanecarboxyliques de formule I:

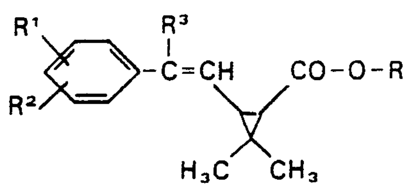

(I)

dans laquelle:

R représente un alcoyle en $C_{1-4}$ ou un radical alcoolique en usage dans les pyréthroïdes,

$R^1$ représente un alcoxy ou alcoylthio, éventuellement substitué par de l'halogène,

$R^2$ représente de l'hydrogène, un alcoxy ou, conjointement avec $R^1$, un alcoylènedioxy éventuellement halosubstitué, et

$R^3$ représente de l'hydrogène ou du chlore,

caractérisé en ce qu'on fait réagir des 1-aryl-4-méthylpenta-1,3-diènes de formule II:

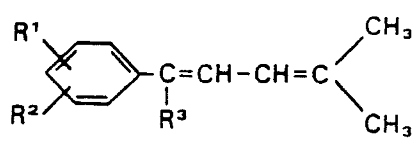

(II)

dans laquelle:

$R^1$, $R^2$, $R^3$ ont la signification indiquée plus haut, avec des esters diazocétiques de formule III:

$$N_2CH-COO-R \quad (III)$$

dans laquelle:

R a la signification indiquée plus haut, en présence de cuivre ou de composé de cuivre à des températures comprises entre 50 et 200° C.

2. 1-Aryl-4-méthylpenta-1,3-diènes de formule II:

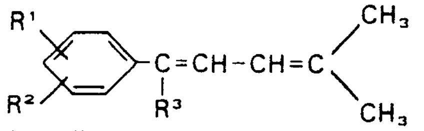
(II)

dans laquelle:

$R^1$ représente un alcoxy ou un alcoylthio, éventuellement substitué par de l'halogène,

$R^2$ représente de l'hydrogène, un alcoxy ou, conjointement avec $R^1$, un alcoylènedioxy éventuellement halosubstitué, et

$R^3$ représente de l'hydrogène ou du chlore.

3. Procédé de préparation de 1-aryl-4-méthylpenta-1,3-diènes de formule II:

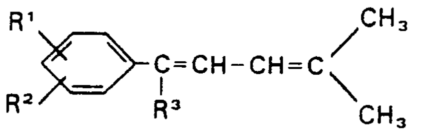
(II)

a) dans laquelle:

$R^1$ représente un alcoxy ou un alcoylthio, éventuellement substitué par de l'halogène,

$R^2$ représente de l'hydrogène, un alcoxy ou, conjointement avec $R^1$, un alcoylènedioxy éventuellement halosubstitué, et

$R_3$ représente de l'halogène,

caractérisé en ce qu'on fait réagir des 3-méthylbutène-2-ylarylcétones de formule IVa:

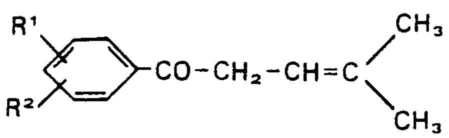
(IVa)

et/ou des 3-méthylbutène-1-ylarylcétones de formule IVb isomères des précédentes:

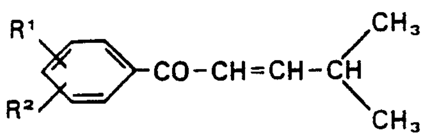
(IVb)

dans laquelle:

$R^1$ et $R^2$ ont la signification indiquée plus haut, avec un pentahalogénure de phosphore, éventuellement avec emploi d'un solvant inerte, à des températures entre −40 et +30° C, puis en ce qu'on traite le mélange de réaction dans le même intervalle de température avec un agent fixateur d'acide, éventuellement en présence d'un catalyseur, ou

b) dans laquelle:

$R^1$ représente un alcoxy ou alcoylthio, éventuellement substitué par de l'halogène,

$R^2$ représente de l'hydrogène, un alcoxy ou, conjointement avec $R^1$, un alcoylènedioxy éventuellement halosubstitué, et

$R^3$ représente de l'hydrogène ou du chlore, caractérisé en ce qu'on fait réagir des esters arylméthanephosphoniques de formule V:

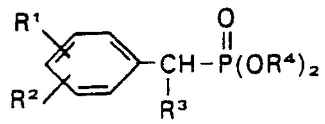
(V)

dans laquelle:

$R^1$, $R^2$ et $R^3$ ont la signification indiquée plus haut, et

$R^4$ représente un alcoyle ou un phényle (ou dans laquelle les radicaux $R^4$ représentent ensemble un alcanediyle à chaîne droite ou ramifiée), avec de la diméthylacroléine de formule VI:

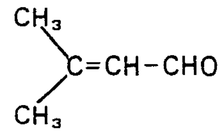
(VI)

en présence d'une base et éventuellement avec utilisation d'un diluant à des températures comprises entre −70 et +150° C, ou

c) dans laquelle:

$R^1$ représente un alcoxy ou alcoylthio, éventuellement substitué par de l'halogène,

$R^2$ représente de l'hydrogène, un alcoxy ou, conjointement avec $R^1$, un alcoylènedioxy éventuellement halosubstitué, et

$R^3$ représente de l'hydrogène ou du chlore, caractérisé en ce qu'on fait réagir des β-arylacroléines de formule VII:

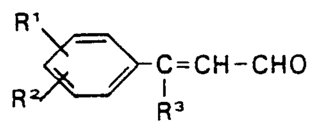
(VII)

dans laquelle:

$R^1$, $R^2$ et $R^3$ ont la signification indiquée plus haut, avec du triphénylisopropylphosphorane de formule VIII:

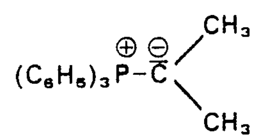
(VIII)

éventuellement avec utilisation de diluants à des températures comprises entre −70 et +50° C, ou

d) dans laquelle:

$R^1$ représente un alcoxy ou alcoylthio, éventuellement substitué par de l'halogène,

$R^2$ représente de l'hydrogène, un alcoxy ou, conjointement avec $R^1$, un alcoylènedioxy éventuellement halosubstitué, et

$R^3$ représente de l'hydrogène ou du chlore, caractérisé en ce qu'on fait réagir des β-arylacroléines de formule VII ci-dessus, dans laquelle $R^1$, $R^2$ et $R^3$ ont la signification indiquée plus haut, avec du chlorure d'isopropylmagnésium de formule IX:

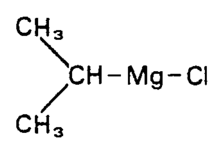
(IX)

éventuellement en présence de diluants à des températures entre −70 et +50° C et en ce qu'on déshydrate ensuite les produits ainsi formés par traitement avec des acides à 0-50° C.